(19) **Europäisches Patentamt / European Patent Office / Office européen des brevets**

(11) **EP 1 047 413 B1**

(12) # FASCICULE DE BREVET EUROPEEN

(45) Date de publication et mention
de la délivrance du brevet:
**03.08.2011 Bulletin 2011/31**

(21) Numéro de dépôt: **99900531.7**

(22) Date de dépôt: **14.01.1999**

(51) Int Cl.:
**A61K 31/185** (2006.01)

(86) Numéro de dépôt international:
**PCT/FR1999/000059**

(87) Numéro de publication internationale:
**WO 1999/036066 (22.07.1999 Gazette 1999/29)**

(54) **COMPOSITION PHARMACEUTIQUE COMPRENANT UN INHIBITEUR SPÉCIFIQUE DE L'AMINOPEPTIDASE A, EN PARTICULIER L'EC33, POUR DIMINUER LA PRESSION ARTÉRIELLE**

PHARMAZEUTISCHE ZUSAMMENSETZUNG, DIE EINEN SPEZIFISCHEN AMINOPEPTIDASE-A INHIBITOR ENTHÄLT, INSBESONDERE EC33, ZUR SENKUNG DES BLUTDRUCKS

PHARMACEUTICAL COMPOSITION COMPRISING A SPECIFIC INHIBITOR OF AMINOPEPTIDASE A, IN PARTICULAR EC33, FOR DECREASING BLOOD PRESSURE

(84) Etats contractants désignés:
**AT BE CH CY DE DK ES FI FR GB GR IE IT LI LU MC NL PT SE**

(30) Priorité: **16.01.1998 FR 9800453**

(43) Date de publication de la demande:
**02.11.2000 Bulletin 2000/44**

(73) Titulaires:
• **INSTITUT NATIONAL DE LA SANTE ET DE LA RECHERCHE MEDICALE (INSERM)**
**75013 Paris (FR)**
• **CENTRE NATIONAL DE LA RECHERCHE SCIENTIFIQUE (CNRS)**
**75794 Paris Cedex 16 (FR)**

(72) Inventeurs:
• **LLORENS-CORTES, Catherine**
**F-91440 Bures sur Yvette (FR)**
• **CORVOL, Pierre**
**F-75015 Paris (FR)**
• **FOURNIE-ZALUSKI, Marie-Claude**
**F-75011 Paris (FR)**
• **ROQUES, Bernard, Pierre**
**F-75014 Paris (FR)**

(74) Mandataire: **Le Coupanec, Pascale A.M.P. et al**
**Cabinet Lavoix**
**2, place d'Estienne d'Orves**
**75441 Paris Cedex 09 (FR)**

(56) Documents cités:
• **CHAUVEL ET AL.: "Differential Inhibition of Aminopeptidase A and Aminopeptidase N by New beta-Amino Thiols" J. MED. CHEM., vol. 37, no. 18, 1994, pages 2950-2957, XP002068832**
• **ZINI ET AL.: "Identification of metabolic pathways of brain angiotensin II and III using specific aminopeptidase inhibitors : Predominant role of angiotensin III in the control of vasopressin release" PROC. NATL. ACAD. SCI. USA, vol. 93, no. 21, 1996, pages 11968-11973, XP002068833**
• **CHAUVEL ET AL.: "Investigation of the Active Site of Aminopeptidase A Using a Series of New Thiol-Containing Inhibitors" J. MED. CHEM., vol. 37, no. 9, 1994, pages 1339-1346, XP002068834**
• **RAMIREZ ET AL.: "Renal aminopeptidase activities in animal models of hypertension" REGULATORY PEPTIDES, vol. 72, no. 2,3, 1997, pages 155-159, XP002068835**
• **AHMAD ET AL.: "Role of Aminopeptidase Activity in the Regulation of the PResor Activity of Circulating Angiotensins" J. PHARMACOL. EXP. THER., vol. 252, no. 2, 1990, pages 643-650, XP002068836**
• **SULLIVAN ET AL.: "Differentail effects of aminopeptidase inhibitors on angiotensin-induced pressor responses" BRAIN RESEARCH, vol. 456, no. 2, 1988, pages 249-253, XP002068837**
• **APPENRODT ET AL.: "Effects of Central Angiotensin II and Angiotensin III on Baroreflex Regulation" NEUROPEPTIDES, vol. 26, no. 3, 1994, pages 175-180, XP002068838**

EP 1 047 413 B1

- **WRIGHT ET AL.: "Aminopeptidase-induced elevations and reductions in blood pressure in the spontaneously hypertensive rat" JOURNAL OF HYPERTENSION, vol. 8, no. 10, 1990, pages 969-974, XP002068839**
- **SONG ET AL.: "Aminopeptidase A antiserum inhibits intracerebroventricular angiotensin II-induced dipsogenic and pressor responses" BRAIN RESEARCH, vol. 744, no. 1, 1997, pages 1-6, XP002068840**
- **ZINI S ET AL: "Aminopeptidase A: Distribution in rat brain nuclei and increased activity in spontaneously hypertensive rats", NEUROSCIENCE, NEW YORK, NY, US, vol. 78, no. 4, 1 April 1997 (1997-04-01), pages 1187-1193, XP009143461, ISSN: 0306-4522, DOI: DOI: 10.1016/S0306-4522(96)00660-4 [retrieved on 1998-02-13]**

Remarques:
  Le dossier contient des informations techniques présentées postérieurement au dépôt de la demande et ne figurant pas dans le présent fascicule.

**Description**

La présente invention se rapporte à une composition pharmaceutique pour son utilisation pour traiter notamment les troubles liés à une hypertension artérielle.

L'hypertension artérielle est une maladie dont les causes sont encore inconnues. Toutefois, on sait que le système nerveux central joue un rôle important dans la régulation du système cardio-vasculaire en contrôlant à la fois l'activité du système nerveux sympathique autonome et du baro-réflexe ainsi que la libération d'hormones hypophysaires.

De même, des travaux cliniques et expérimentaux suggèrent que l'activité du système nerveux central et des nerfs sympathiques périphériques participent à la genèse de l'hypertension artérielle.

Il a également été montré qu'un système rénine-angiotensine existait en outre dans le système nerveux central. Il s'avère qu'il contrôle les fonctions cardio-vasculaires et l'homéostasie des fluides corporels. Tous les composants du système rénine-angiotensine systémique, y compris les précurseurs et enzymes nécessaires à la formation et la dégradation des angiotensines, de même que les récepteurs d'angiotensine ont déjà été identifiés à l'intérieur du cerveau.

Dans le système rénine-angiotensine systémique, on sait en particulier que l'angiotensine II est générée sous l'action d'une enzyme essentiellement membranaire appartenant au groupe des métalloprotéases à zinc. Cette ectopeptidase est connue sous le nom d'enzyme de conversion de l'angiotensine (ECA) car elle transforme le peptide inactif angiotensine I en angiotensine II.

Il s'avère que cette angiotensine II est transformée in vivo en angiotensine III (AngIII) sous l'action d'une autre ectopeptidase à zinc, récemment clonée, l'aminopeptidase A (APA), qui élimine le résidu aspartyl N-terminal de l'angiotensine II pour conduire à l'angiotensine III. Cette angiotensine III est elle-même détruite par diverses peptidases dont notamment l'aminopeptidase N (APN).

Ces deux ectopeptidases à zinc, APA et APN appartiennent au groupe d'enzymes de type thermolysine et possèdent une homologie significative entre leurs séquences d'acides aminés.

Il a été démontré que l'inhibition de l'enzyme de conversion de l'angiotensine I en angiotensine II, ECA, présente dans le système rénine-angiotensine systémique, conduit via un blocage de la formation de l'angiotensine II, à une baisse de la pression artérielle particulièrement sensible chez les personnes souffrant d'hypertension. Il s'avère que ces inhibiteurs bloquent l'ECA périphérique à la fois dans la circulation et surtout dans de nombreux tissus : endothélium vasculaire, poumon, rein, etc.

En conséquence, jusqu'ici il était proposé que l'angiotensine II était le médiateur principal du système rénine-angiotensine cérébral par analogie au système périphérique.

L'article Zini et al. (Proc. Natl. Acad. sci. USA, 93, 11968-11973, 1996) a montré l'implication de l'aminopeptidase A, in vivo, dans le métabolisme de l'angiotemine II cérébrale.

L'article song et al. (brain Research, 744: 1-6, 1997) a décrit l'effet d'un antisérum anti-APA et anti-APN sur l'effet presseur induit par l'administration entracérétroventriculaire d'angiotenne II exogène.

En fait, contrairement à ce qu'il était admis, il semble que dans le système rénine-angiotensine cérébral, l'étape critique ne serait pas la formation de l'angiotensine II par action de l'ECA sur l'angiotensine I mais la formation de l'angiotensine III par action de l'APA sur l'angiotensine II. Ce rôle déterminant de l'angiotensine III dans le système rénine-angiotensine cérébral est notamment conforté par les résultats figurant dans l'exemple 4 ci-après.

L'ensemble de ces données tendent donc à identifier l'angiotensine III comme le peptide effecteur du système rénine-angiotensine cérébral, responsable de l'augmentation de la pression artérielle. Plus précisément, dans le cerveau, il apparaît que l'Ang III exerce un effet stimulateur tonique sur le contrôle central de la pression artérielle.

La présente invention repose précisément sur la mise en évidence que l'angiotensine III joue un rôle essentiel dans le contrôle de la pression artérielle au niveau central

Plus particulièrement, la présente invention vise à proposer une composition pharmaceutique permettant de diminuer la pression artérielle et donc de s'opposer à une augmentation de la pression induite par l'angiotensine III.

Plus précisément, elle décrit une composition pharmaceutique utile pour diminuer la pression artérielle, caractérisée en ce qu'elle comprend à titre de principe actif au moins un inhibiteur sélectif de l'aminopeptidase A.

Comme évoqué précédemment, l'aminopeptidase A (APA) est une ectoenzyme appartenant à la famille des métalloprotéases à zinc et dont la thermolysine constitue le modèle bactérien.

L'APA est une glycoprotéine qui se présente sous la forme d'un homodimère.

Le clonage de son ADNc a révélé que chaque monomère est composé d'un domaine d'ancrage séparant un court segment cytosolique N-terminal d'un large domaine extracellulaire C-terminal qui contient le site actif dont plus particulièrement le site liant le zinc.

Or, il s'avère que l'APA présente 34 % d'identité de séquence en acides aminés avec l'APN, impliquée pour sa part dans la dégradation de l'angiotensine III. Cette homologie est par ailleurs la plus élevée au niveau précisément du site actif présent dans le domaine extracellulaire glycosylé.

Il est clair que cette homologie de séquence entre l'APA et l'APN constitue un handicap pour obtenir des inhibiteurs spécifiques et sélectifs à l'égard de l'APA.

Comme évoqué ci-dessus, l'inhibiteur mis en oeuvre dans la composition décrite est un inhibiteur sélectif à l'égard de l'APA. Cette sélectivité se traduit notamment par une affinité multipliée environ d'au moins un facteur 100 pour l'APA comparativement à l'APN.

La présente invention revendique en particulier une composition pharmaceutique caractérisée en ce qu'elle comprend à titre de principe actif au moins l'acide (S) 3-amino-4-mercaptobutylsulfonique ou l'un de ses sels avec un acide ou une base pharmaceutiquement acceptable pour son utilisation en tant que médicament.

La présente invention revendique également un inhibiteur sélectif de l'aminopeptidase A qui :

- possède une affinité multipliée d'au moins un facteur 100 pour l'aminopeptidase A comparativement à l'aminopeptidase N, ou
- possède un pouvoir inhibiteur sur l'aminopeptidase A in vitro inférieur ou égal à $10^{-7}$ M
  associé le cas échéant à un véhicule pharmaceutiquement acceptable, pour son utilisation en tant que médicament pour diminuer la pression artérielle.

Plus précisément, est considérée selon la présente demande comme un inhibiteur sélectif à l'égard de l'APA, une molécule qui répond au moins à l'un des critères suivants :

- son pouvoir inhibiteur sur l'APA *in vitro* est inférieur ou égal à $10^{-7}$ M,
- elle présente un facteur de sélectivité d'environ 100 par rapport aux enzymes aminopeptidase N, aminopeptidase B (EC 3.4.11.6), endopeptidase neutre (EC 3.4.24.11), et l'enzyme de conversion de l'angiotensine (EC 3.4.15.1).
- injectée *in vivo* par voie intracérébroventriculaire ou systémique (si elle traverse la barrière hématoencéphatique), elle bloque la formation de l'angiotensine III.

Comme inhibiteur de l'APA convenant tout particulièrement à l'invention, on peut notamment citer l'acide (S) 3-amino-4-mercaptabutylsulfonique ou l'un de ses sels avec un acide ou une base pharmaceutiquement acceptable.

Comme il ressort des exemples présentés ci-après, le (S) 3-amino 4-mercaptobutylsulfonate de sodium, désigné ci-après sous le symbole EC33, présente une activité inhibitrice significative à l'égard de l'APA.

Cet inhibiteur présente avantageusement un facteur de sélectivité de 100 par rapport à l'aminopeptidase N.

Injecté chez la souris par voie intracérébroventriculaire, à raison de 30 μg, on note une augmentation significative de la demi-vie de l'angiotensine II (30 μg), d'un facteur de l'ordre de 2,6 comparativement à celle observée chez un animal témoin. Parallèlement, il bloque totalement la formation de l'angiotensine III au niveau de l'hypothalamus.

De même, des expériences réalisées chez des rats normotendus (WKY) ou hypertendus (SHR) montrent que l'injection de l'EC33 permet de diminuer significativement la pression artérielle. L'effet hypotenseur de cet inhibiteur de l'APA, l'EC33, est maximal pour une dose de 100 μg. Il est de -22 mmHg chez le rat normotendu et de -28 mmHg chez le rat hypertendu. La durée d'action à cette dose est en moyenne entre 40 et 60 minutes.

Ces résultats montrent donc que l'APA, l'enzyme responsable de la production d'angiotensine III dans le système nerveux central constitue une nouvelle cible thérapeutique du système rénine-angiotensine cérébral et que l'utilisation d'un inhibiteur de l'APA permet de réduire significativement la pression artérielle.

Les compositions pharmaceutiques revendiquées peuvent éventuellement contenir un ou plusieurs véhicules pharmaceutiquement acceptables. Ces véhicules sont choisis de manière à constituer une forme pharmaceutique administrable de façon classique par voie orale, transmuqueuse, parentérale ou rectale.

Leurs modes d'administration, posologies et formes galéniques optimaux peuvent être déterminés selon les critères généralement pris en compte dans l'établissement d'un traitement thérapeutique adapté à un patient comme par exemple l'âge ou le poids corporel du patient, la gravité de son état général, la tolérance au traitement et les effets secondaires constatés, etc.

En conséquence, les compositions selon l'invention sont particulièrement intéressantes pour traiter l'hypertension artérielle essentielle au cours de laquelle l'hyperactivité sympathique observée souvent durant la phase précoce, est vraisemblablement médiée par une activité accrue du système rénine-angiotensine cérébral.

A titre illustratif mais non limitatif des troubles susceptibles d'être traités avec les compositions revendiquées, on peut notamment citer les insuffisances cardiaque et rénale, les troubles de l'homéostasie hydrodynamique et la réduction de la protéinurie chez les diabétiques.

En outre, les compositions selon l'invention peuvent être avantageusement utilisées en complément des bloqueurs du système rénine-angiotensine systémique.

A titre représentatif de ces bloqueurs, on peut notamment citer les inhibiteurs de l'enzyme de conversion tel que l'Enaprilate et les antagonistes des récepteurs de l'angiotensine II comme le Losartan.

Selon une variante de l'invention, la composition revendiquée comprend en outre un inhibiteur de l'enzyme de conversion de l'angiotensine I ou un antagoniste des récepteurs AT1.

Ce type de bloqueurs du système rénine-angiotensine systémique s'avère efficace dans des pathologies comme l'hypertension, l'insuffisance cardiaque congestive, la dysfonction ventriculaire gauche après infarctus du myocarde, mais également dans la régression de la protéinurie chez les diabétiques et dans la réduction de la progression de l'insuffisance rénale chronique.

En agissant également au niveau du contrôle central de la pression artérielle et en bloquant l'activité du système rénine-angiotensine central via leur inhibiteur d'APA, les compositions revendiquées seront également efficaces pour traiter les infections évoquées ci-dessus.

La demande décrit également à l'utilisation d'un inhibiteur d'APA tel que défini ci-dessus, associé le cas échéant à un véhicule pharmaceutiquement acceptable pour la fabrication d'un médicament utile pour diminuer la pression artérielle.

Il peut notamment s'agir de l'acide (S) 3-amino 4-mercaptobutylsulfonique ou l'un de ses sels, pharmaceutiquement acceptables avec un acide ou une base.

Figures

Figure 1 : Evolution au cours du temps de la teneur en AngII tritiée dans l'hypothalamus murin en présence ou non de l'EC33.

Figure 2 : Représentation semilogarithmique des teneurs en AngII tritiée dans l'hypothalamus murin en absence (contrôle) ou en présence d'EC33 (30 μg).

Figure 3 : Effet de différentes doses d'EC33 injectées par voie intracérébroventriculaire sur la pression artérielle chez des rats SHR et WKY.

Figure 4 : Effet de différentes doses d'EC33 en terme de durée de l'effet sur la pression artérielle chez des rats SHR et WKY.

Figure 5 : Effet presseur d'un inhibiteur de l'APN, le PC18, chez les rats SHR.

Figure 6 : Effet en terme de durée d'un inhibiteur de l'APN, le PC18, sur la pression artérielle, chez le rat SHR.

Figure 7 : Effet presseur d'un inhibiteur de l'APN, le PC18, chez le rat normotendu en présence d'un antagoniste des récepteurs angiotensinergiques de type 1, AT1.

Figure 8 : Effet sur la pression artérielle de l'inhibiteur EC33 injecté par voie intracérébroventriculaire (i.c.v.) ou par voie intraveineuse (i.v.) chez des rats vigiles normotendus (WKY) ou spontanément hypertendus (SHR).

## MATERIELS ET METHODES

### A. Composés :

L'EC33, (S)-3-amino-4-mercapto-butane sulfonate de sodium) est préparé selon les protocoles décrits dans Chauvel et al. J. Med. Chem. 37, 1339-1346 et 2950-2957.

Le PC 18, méthioninethiol est un inhibiteur sélectif et de haute affinité pour l'APN (Ki = 9 nm) Il est préparé selon le protocole décrit dans Fournié-Zaluski et al., J. Med. Chem. 35, 1259-1266.

Les différentes formes d'angiotensine II et III considérées ci-après sont disponibles auprès des sociétés Amersham ou Sigma.

### B. Animaux:

Des souris mâles suisses (disponibles auprès de l'Institut Iffa Credo) pesant 18 à 20 grammes sont maintenues sous lumière artificielle (12 heures en phase éclairée - 12 heures dans la pénombre) et disposent d'aliments et d'eau ad libitum. Les expériences sont réalisées entre 9 h et 11 h dans la matinée. Les drogues sont administrées dans le ventricule latéral par voie intracérébroventriculaire (I.C.V.) à un taux de 5μl par souris selon la méthode de Haley et McCormick (Br. J. Pharmacol. 12, 12-15).

Les rats utilisés sont des rats mâles Sprague-Dawley, pesant 200 à 250 grammes, certains d'entre eux sont des rats âgés de 11 semaines (SHR). Des rats normotendus Wistar Kyoto sont également utilisés à titre de contrôle. Les animaux sont conservés pendant une semaine avec des périodes de luminosité et de pénombre de 12 heures alternées, et disposent d'eau et d'aliments ad libitum. Pour chaque expérience les rats SHR et WKY sont pesés et leur pression artérielle et leur rythme cardiaque sont enregistrés en continu par voie invasive après la pose d'un cathéter (PE 50) dans l'artère fémorale. Le cathéter est relié à un capteur de pression (COBE) et connecté à un conditionnement de signaux relié à un amplificateur. Le système informatique comprend un système d'interface MacLab et un logiciel (Chart et Scope) d'application qui permet l'acquisition et le calcul des données par l'intermédiaire d'un ordinateur MacIntosh.

**C. Modes d'administration et méthodes d'analyse :**

- Injection intracérébroventriculaire

Pour chaque expérience, six à huit souris sont utilisées pour chacune des conditions. Un mélange de 2 $10^6$ cpm [$^3$H]Ang II et 30 $\mu$g d'Ang II non marqués est administré dans un volume de 5 $\mu$l, conjointement ou non avec l'EC33 (30 $\mu$g). Les inhibiteurs sont dissous dans une solution saline isotonique ajustée à un pH de 7,4 avec une solution de NaOH à 0,01 M. A différents temps après l'injection, les souris sont décapitées, leurs cerveaux prélevés et leurs hypothalamus disséqués sur la glace et homogénéisés par sonication (ultrasons, Anemasse, France) dans 10 volumes d'une solution à 0,1 M d'HCl refroidie dans de la glace. L'homogénéat est centrifugé à raison de 12 000 x g x 20 min. à 4 °C et le surnageant est conservé à -80 °C jusqu'à l'analyse.

- Séparation des angiotensines II et III par chromatographie échangeuse d'ions.

Les quantités respectives en angiotensines II et III marquées présentes dans l'hypothalamus sont déterminées selon le protocole de Ledwith et al. (Anal. Biochem. 213, 349-355). Pour ce faire, un gel de sépharose à flux rapide (1/2 de volume mort, Pharmacia) est chargé dans des colonnes seringues de 3 ml et équilibré à température ambiante avec 15 ml d'un tampon (10 mM acétate de sodium-50 mM NaCl, pH 5,0). Après avoir été décongelés, les extraits d'hypothalamus contenant environ 30 000 cpm sont dilués dans 2 ml de tampon d'équilibration, chargés sur les colonnes et lavés avec 1 ml de tampon. Une première fraction (F1) contenant de l'angiotensine II tritiée et d'autres métabolites de l'angiotensine tritiée est éluée avec 7 ml d'une solution de 10 mM d'acétate de sodium/80 mM NaCl/5 % d'acétonitrile à pH 5. Une seconde fraction (F2) contenant de l'angiotensine III tritiée seule est éluée avec 3 ml d'une solution HCl 3 M. Les fractions F1 et F2 sont ensuite analysées par HPLC pour déterminer leur teneur respective en angiotensine II tritiée et en angiotensine III tritiée.

- Concentration par passage sur colonne C$_{18}$Sep-pak®.

Dans la perspective d'une analyse HPLC, les fractions F1 et F2 sont initialement concentrées par extraction sur un dispositif C$_{18}$Sep-pak® Cartridges (Waters). Pour cela, de l'acide trifluoroacétique (1 % à concentration finale) est ajouté à la fraction F1 qui est ensuite chargée sur Sep-pak®, équilibrée au préalable avec une solution aqueuse en acide trifluoroacétique à 1 %. La fraction radioactive est éluée avec 1,5 ml d'acétonitrile à 100 %. Sous ces conditions on récupère 75 % en angiotensine II. La fraction F2 est chargée sur Sep-pak® équilibrée au préalable avec 2 ml d'une solution de méthanol à 100 % suivie par 5 ml d'eau. La fraction radioactive est éluée avec 1,5 ml de méthanol à 100 %. On récupère ainsi 92 % en angiotensine III. Après élution sur Sep-pak®, les fractions F1 et F2 respectives sont lyophilisées, dissoutes dans 0,2 ml d'une solution d'acide acétique à 0,01 M avec 20 ng d'angiotensine II et d'angiotensine III à titre d'étalon interne et analysées par HPLC.

- Analyse HPLC.

L'analyse HPLC est réalisée en utilisant une colonne à phase inverse H Hypersil ODS®-3 $\mu$m (Shandon, Pittsburgh) chauffée à 45 °C puis avec une élution isocratique à un flux de 0,6 ml par minute. La phase mobile consiste en H$_3$PO$_4$ 86 mmolaire, ajusté à pH 3 avec de la triéthylamine, et en 17,5 % d'acétonitrile. Après injection de l'échantillon (0,15 ml), des fractions de 0,10 ml sont collectées pendant 12 minutes. Leur teneur en radioactivité est estimée avec un compteur béta. Dans de telles conditions, les temps de rétention des angiotensines témoins sont de 9,5 minutes pour l'angiotensine II et 7,8 minutes pour l'angiotensine III.

**D. Mesure de l'activité APA *in vitro* :**

Le dosage de l'activité APA est basé sur le protocole de Goldbarg adapté à l'échelle de dosage sur microplaques (Pro Bind™ 3915) (Chauvel et coll., 1994).

- Principe

*In vitro*, en présence d'ions calcium, l'APA hydrolyse l'$\alpha$-L-glutamyl-$\beta$-naphtylamide (glu$\beta$Na) en glutamate et $\beta$-naphtylamine ($\beta$Na). Une réaction de diazotation en milieu acide permet de révéler le $\beta$-naphtylamine par formation d'un complexe de couleur violette une mesure de spectrophotométrie permet alors de connaître la quantité de complexe formé et, par référence à une gamme étalon réalisée avec des concentrations croissantes de $\beta$-naphtylamine, d'en déduire l'activité enzymatique de l'échantillon.

- Réactifs

Le substrat GluβNa et le β-naphtylamine (Bachem) sont solubilisés dans le DMSO (diméthyl sulfoxyde) et l'HCl 0,1 N respectivement, et conservés à -20°C à la concentration de $10^{-2}$ M. La réaction de diazotation se fait en présence de nitrite de sodium (87mM), de sulfamate d'ammonium (130 mM) et du N-(1-naphtyl)éthylènediamine dihydrochloride ( 23 mM).

- Réaction enzymatique

La réaction a lieu à pH 7,4 en tampon Tris-HCl 50 mM, en présence de calcium ($CaCl_2$, 4 mM) ; l'échantillon à doser est incubé à 37°C en présence de substrat (GluβNa, 200 µM) et en absence ou en présence de différentes concentrations de l'inhibiteur à tester dans un volume final de 100µl. La réaction est arrêtée par addition de 10 µl d'HCl 3N. Une gamme étalon de β-naphtylamine en milieu acide (ajouter 10 µl d'HCl 0,1 N) est effectuée en parallèle.

- Révélation du produit formé

On ajoute dans chaque puits :

- 25 µl de nitrite de sodium (mélanger, attendre 5 minutes à température ambiante),
- 50 µl de sulfamate d'ammonium (agiter, attendre 5 minutes à température ambiante),puis
- 25 µl de N-(1-naphtyl)éthylènediamine dihydrochloride 23 mM (mélanger, attendre la stabilisation de la couleur violette environ 30 minutes à 37°C).

L'absorbance est ensuite mesurée à 540 nm.

**E. Mesure de l'activité APN**

Le principe du dosage repose sur l'hydrolyse de la [3H] Leu-enképhaline (10 nM) par l'APN en deux métabolites, la [3H] Tyr et le tétrapeptide Gly-Gly-Phe-Leu. Une fois la réaction arrêtée, le métabolite [3H] est isolé sur colonne de Porapak et sa radioactivité est estimée par scintillation liquide. L'activité de l'APN est estimée en fmoles de substrat hydrolysé/min/mg de protéine.

**F. Estimation du pouvoir inhibiteur de l'EC33 et du PC18**

Le pouvoir inhibiteur de l'EC33 et du PC18 sur l'APA ou l'APN est estimé en mesurant l'activité enzymatique de l'APA ou l'APN purifiées en présence de concentrations croissantes de l'inhibiteur. A partir de ces données, la concentration efficace 50 est calculée d'après un programme de régression non linéaire des moindres carrés Graph Pad PRISM™, version 2), ce qui permet de déduire le KI de ces molécules d'après la formule suivante :

$$KI = \frac{IC50}{1 + \dfrac{Km}{[S]}}$$

Le Km de l'APA pour le GluβNa est de 100 µM (Vazeux et coll., J. biochem., 1996, vol. 271 :9069-9074).
Le Km de l'APN pour la Leu-enképhaline est de 50 µM (Chauvel et coll., J. Med. Chem., 1994, vol. 37.2950-2956).

**EXEMPLE 1 :**

**Test *in vitro* du pouvoir inhibiteur de l'EC33 et du PC18 sur l'APA et l'APN.**

Ces pouvoirs inhibiteurs sont estimés conformément au protocole décrit dans le chapitre "matériels et méthodes".
L'EC33 présente un KI de 2,5±0,6. 10-7 M pour l'APA et un facteur de sélectivité d'environ 100 par rapport à l'APN (25±11, 10-6 M).

Le pouvoir inhibiteur du PC 18 sur l'APN est de $1,1 \pm 0,1$ 10-8 M, il est 1000 fois moins actif sur l'APA ($11 \pm 1,7$, 10-6 M).

## EXEMPLE 2 :

### *Effet in vivo* de l'inhibiteur EC33 sur le métabolisme des angiotensines cérébrales AngII et AngIII présentes dans l'hypothalamus murin.

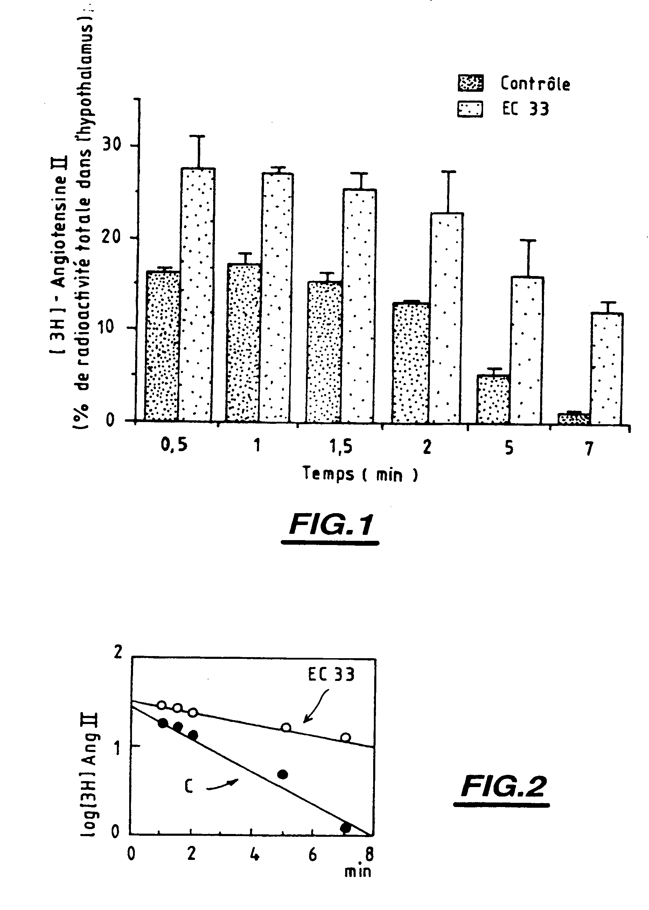

Les cinétiques d'apparition et de disparition dans l'hypothalamus des angiotensines II et III radiomarquées sont déterminées après avoir injecté chez la souris par voie intracérébroventriculaire une quantité connue d'AngII tritiée ($2.10^6$ cpm) avec 30 $\mu$g d'AngII non radioactive, en présence ou en absence de l'inhibiteur (EC33, 30 $\mu$g). Les taux de peptides tritiés dans les tissus sont quantifiés selon le protocole décrit dans le chapitre Matériels et méthodes par HPLC.
L'évolution de la teneur en AngII [3H] dans l'hypothalamus après injection i.c.v. d'AngII [3H], en absence ou en présence de l'EC33 est reportée en figure 1.
Chez la souris témoin, la teneur en AngII [3H] décroît rapidement après 1,5 minutes et n'est plus détectable au delà de 7 minutes. En présence de l'EC33, cette teneur en AngII [3H] est significativement augmentée entre 0,5 et 7 minutes pour atteindre environ 11 fois la teneur de l'essai témoin.
Une représentation sémilogarithmique du taux de disparition en teneur AngII [3H] en présence de l'EC33 par rapport au contrôle est présentée en figure 2.
On note que chez l'animal traité avec EC33, la demi vie de l'AngII[3H], ($5,38 \pm 0,21$ min) est augmentée d'un facteur 2,6 comparativement à celle d'un animal témoin.
Chez la souris témoin, la formation d'AngIII tritiée est maximale à 1 minute et les taux diminuent progressivement jusqu'à 10 minutes. En présence d'EC 33, la formation d'AngIII tritiée est immédiatement bloquée et des taux très faibles sont mesurés tout au long de l'expérience.

## EXEMPLE 3 :

### Effet *in vivo* de l'inhibiteur EC33 sur une augmentation de pression artérielle induite par AngII.

Ces expériences sont effectuées chez le rat normotendu Wistar Kyoto (WKY) ou spontanément hypertendu (SHR âgé de 12 semaines).
Les animaux sont anesthésiés à l'Inactine (5-éthyl-2-(1'méthylpropyl)-2-thiobarbiturale) anesthésique utilisé régulièrement dans les expériences de physiologie rénale ou cardiovasculaire. Ses caractéristiques sont d'entraîner une très faible baisse de la pression artérielle, visible uniquement chez le rat SHR, et surtout de permettre le maintien d'une pression artérielle de base stable tout au long de l'expérience.
La pression artérielle moyenne (systolique + diastolique) est mesurée en continu, par voie invasive, après cathétérisme de l'artère fémorale. Les peptide ou les inhibiteurs sont injectés par voie intracérébroventriculaire (i.c.v.) à l'aide d'une canule placée dans le ventricule latéral, sous stéréotaxie (Atlas stéréotaxique ; G. Paxinos et C. Watson, Academic Press, 1986).
Les expériences sont réalisées sur des groupes de 4 à 20 animaux analysés individuellement. Les résultats sur des lots d'animaux témoins et traités ont été comparés à l'aide d'une analyse de variance (ANOVA) suivi d'un test de Student non apparié.
Il est procédé à une injection d'angiotensine II associée, le cas échéant, à un injection consécutive d'EC33.
Les essais sont réalisés selon les schémas ci-après qui rendent également compte des résultats obtenus.

# WKY

AngII 10 ng

Sérum phy.

Pression artérielle basale (mmHg)

$122 \pm 1$    5 min    $122 \pm 1$    10 min    $\Delta PA = +9.3 \pm 1.1^*$    $122 \pm 1$

$t = 8 \pm 1.6$ min

EC 33 100 µg

AngII 10ng

Pression artérielle basale (mmHg)

$120 \pm 2$    5 min    $\Delta PA1 = -21.8 \pm 1.3$    $\Delta PA2 = -21.8 \pm 1.3$

15 min

# SHR

AngII 10 ng

Sérum phy.

Pression artérielle basale (mmHg)

$166 \pm 4$    5 min    $166 \pm 4$    5 min    $\Delta PA = +9,3 \pm 3,1^*$    $166 \pm 4$

$t = 6 \pm 2,4$ min

EC 33 100 µg

AngII 10ng

Pression artérielle basale (mmHg)

$170 \pm 3$    5 min    $\Delta PA1 = -24,3 \pm 4,6^*$    $\Delta PA2 = -24,3 \pm 4,6$   NS

15 min

On note que l'AngII à une dose de 10 ng augmente significativement la pression artérielle (PA). L'effet étant légèrement plus important chez le rat SHR que chez le rat WKY. L'amplitude maximale de l'effet est d'environ 10 mm Hg pour 10 ng d'AngII et la durée d'action se situe entre 7 et 9 minutes.

Lorsque l'AngII (10 ng) est injectée en présence de 100 µg de l'inhibiteur d'aminopeptidase A, l'EC33, l'effet presseur de l'AngII est complètement aboli.

La spécificité d'action de l'EC33 a également été démontrée par son inefficacité à modifier l'effet presseur de l'AngIII (30 ng). Les schémas figurant ci-après rendent compte des résultats obtenus.

## WKY

## SHR

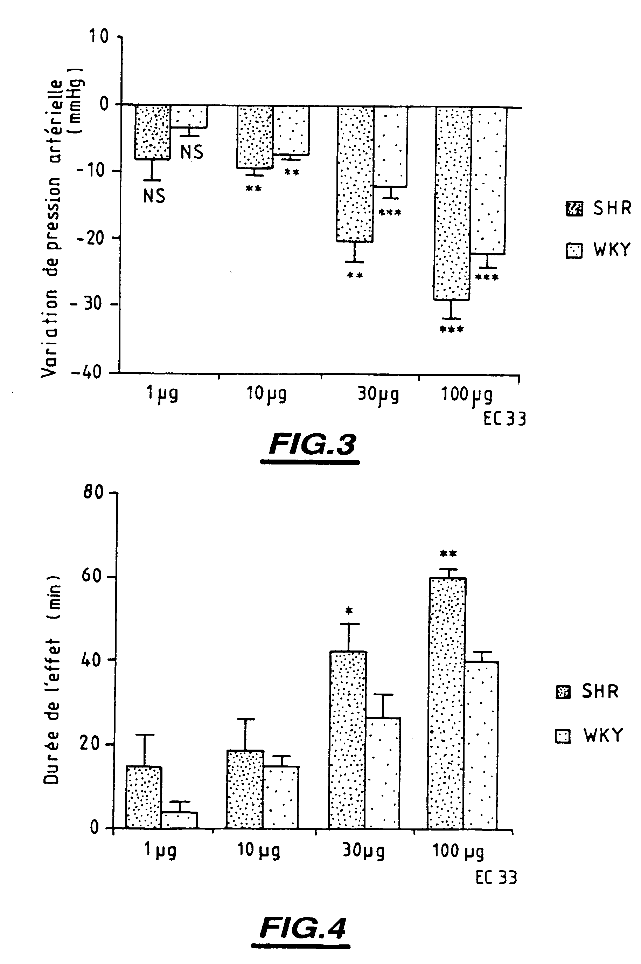

L'effet dose dépendante de l'EC33 injecté seul a également été apprécié sur les plans durées d'action et effet hypotenseur. Les résultats obtenus sont représentés en figures 3 et 4.

De manière générale, lorsque l'on bloque l'APA par l'EC33, on observe une baisse de PA dose dépendante, plus marquée chez le rat hypertendu que chez le rat normotendu. L'effet hypotenseur de l'inhibiteur de l'APA est maximal pour une dose de 100 $\mu$g. Il est de -22 mm Hg chez le rat normotendu et de -28 mm Hg chez le rat hypertendu.

La durée d'action à cette dose est en moyenne entre 40 et 60 minutes.

En conséquence, ces données indiquent que dans les conditions basales, l'angiotensine III endogène exerce un effet tonique positif sur le contrôle central de la pression artérielle.

**EXEMPLE 4 :**

**Démonstration du rôle déterminant de l'AngIII dans le système rénine-angiotensine cérébral.**

Cet essai est effectué à l'aide d'un inhibiteur de l'aminopeptidase N (APN), le PC18 (3- amino-4-mercaptométhionine). Cette APN est responsable de la dégradation de l'angiotensine III en angiotensine IV.

Conformément au protocole décrit en exemple 3, on injecte l'AngIII (10 ng) en présence de 60 µg, de PC18 chez le rat SHR. A titre d'essai témoin, il est procédé à une injection de PC18 seul.

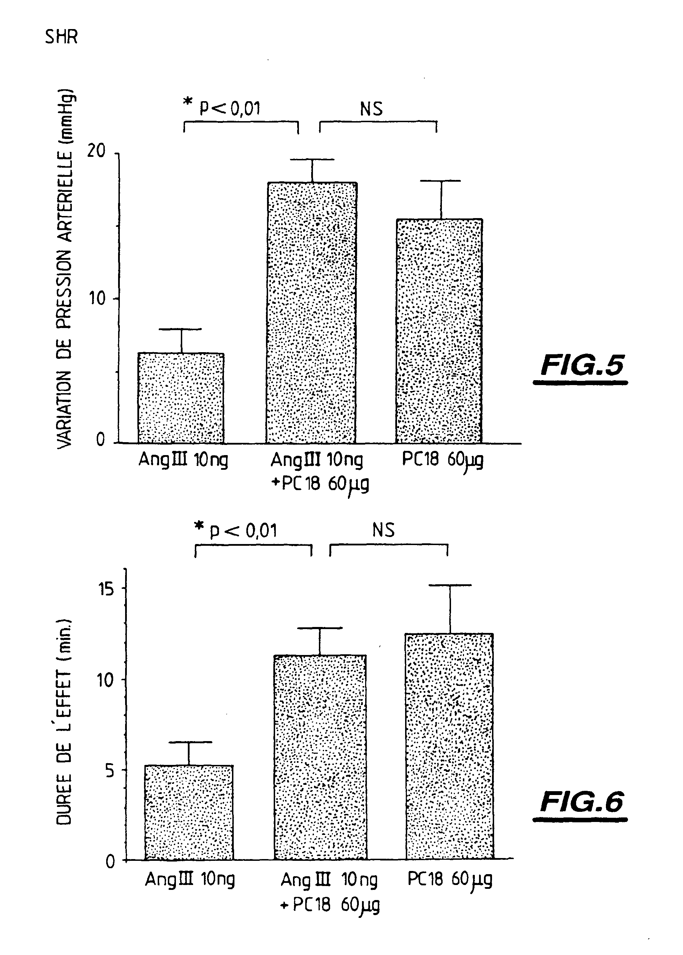

L'effet de cette co-injection est appréciée en terme de pression artérielle et de durée et les résultats obtenus sont présents en figures 5 et 6.

On observe une potentialisation de l'effet presseur de l'Ang III (10 ng) en prenant de l'inhibiteur PC18 (60 µg). A 60 µg, le PC18 seul augmente chez le rat SHR, la pression artérielle de 15 mm Hg et l'effet dure environ 12 minutes.

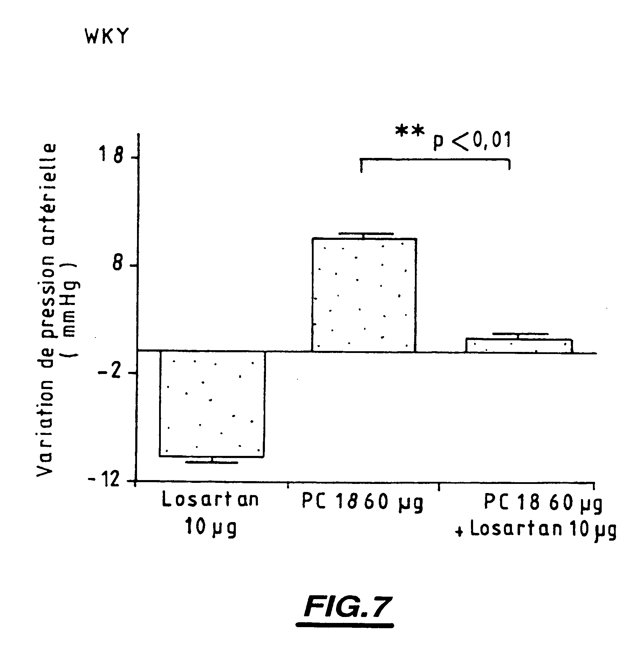

Par ailleurs, il a également été montré chez le rat normotendu ou hypertendu, que l'augmentation de pression artérielle (+ 10 ou + 13 mm Hg respectivement) induite par 60 µg de PC18 est antagonisée par un prétraitement avec un antagoniste de récepteurs angiotensinergiques de type 1 (AT1), le Losartan (10 µg). Ceci est en particulier illustré en figure 7 pour le rat normotendu.

Cet effet antagoniste est de l'ordre de 85 % chez le rat normotendu et de 52% chez le rat hypertendu.

De ces données, il ressort que l'effet presseur du PC18 est dû à une accumulation de l'AngIII endogène via les récepteurs AT1.

**EXEMPLE 5 :**

**Effet sur la pression artérielle de l'inhibiteur EC33 injecté par voie intracérébroventriculaire (i.c.v.) ou par voie intraveineuse (i.v.) chez des rats vigiles.**

Ces expériences sont effectuées chez le rat SHR âgé de 12 semaines (300-350 g). Pendant l'opération, les animaux sont anesthésiés ponctuellement (3 heures) avec du pentobarbital sordique (50 mg/kg intrapéritonéal, Laboratoire Sentravet, Plancoët, France). Les expériences sont réalisées au minimum 24 heures après l'opération.

- injection par voie i.c.v.

Les injections sont réalisées à l'aide d'une canule placée dans le ventricule latéral, sous stéréotaxie et la pression artérielle est mesurée en continu par voie invasive après cathétérisme de l'artère fémorale. Le cathéter est extériorisé au niveau de la nuque de l'animal et relié à un système de fixation permettant au rat de se mouvoir librement dans sa cage.

- injection par voie i.v.

Un premier cathéter est placé dans la veine fémorale afin de réaliser les injections, un second cathéter est introduit dans l'artère fémorale afin de pouvoir mesurer la pression artérielle en continu. Les deux catéthers sont ensuite extériorisés comme indiqué ci-dessus.

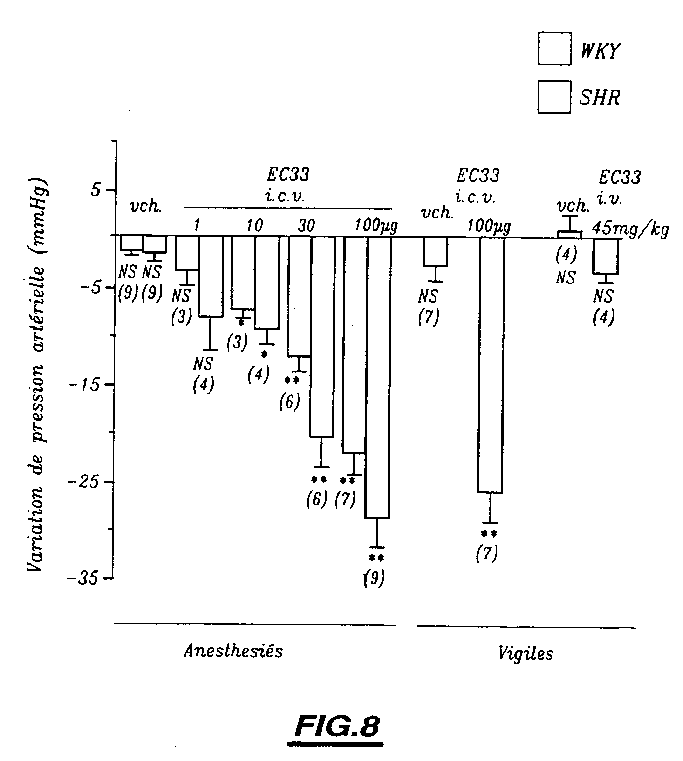

Les résultats obtenus sont représentés en figure 8.

On note que chez les rats SHR vigiles, l'injection i.c.v. de l'EC33 à une dose de 100 µg diminue significativement la pression artérielle (-26 mmHg) du même ordre que chez les animaux anesthésiés (-28 mmHg) (Fig. b). Cet effet hypotenseur dure cependant moins longtemps (40 min).

En revanche, injectée par voie i.v., l'EC33 à une dose de 45 mg/kg n'induit pas d'effet hypotenseur significatif chez les rats SHR vigiles (Fig. c).

Ces résultats montrent donc que l'AngIII cérébrale endogène exerce une action tonique dans la régulation de la pression artérielle aussi bien chez le rat anesthésié que chez le rat vigile. Les résultats obtenus à la périphérie montrent que le bloquage de la conversion de l'AngII circulante en AngIII n'induit pas de modification significative de la pression artérielle.

Ceci suggère que l'APA cérébrale joue un rôle prépondérant dans la régulation de la pression artérielle comparée à l'APA périphérique qui semble avoir une contribution moins importante.

**Revendications**

**1.** Composition pharmaceutique **caractérisée en ce qu'**elle comprend à titre de principe actif au moins l'acide (S) 3-amino-4-mercaptobutylsulfonique ou l'un de ses sels avec un acide ou une base pharmaceutiquement acceptable pour son utilisation en tant que médicament.

**2.** Composition pour son utilisation selon la revendication 1 **caractérisée en ce qu'**elle comprend en outre au moins un véhicule pharmaceutiquement acceptable.

**3.** Composition pour son utilisation selon la revendication 1 ou 2, **caractérisée en ce qu'**elle comprend en outre un inhibiteur de l'enzyme de conversion de l'angiotensine I ou un antagoniste des récepteurs AT1.

**4.** Inhibiteur sélectif de l'aminopeptidase A qui :

- possède une affinité multipliée d'au moins un facteur 100 pour l'aminopeptidase A comparativement à l'aminopeptidase N, ou
- possède un pouvoir inhibiteur sur l'aminopeptidase A in vitro inférieur ou égal à $10^{-7}$M, associé le cas échéant à un véhicule pharmaceutiquement acceptable, pour son utilisation en tant que médicament pour diminuer la pression artérielle.

**5.** Inhibiteur pour son utilisation selon la revendication 4 , dans lequel l'inhibiteur est l'acide (S) 3-amino-4-mercaptobutylsulfonique ou l'un de ses sels avec un acide ou une base pharmaceutiquement acceptable.

**6.** Inhibiteur pour son utilisation selon la revendication 4, ou 5, dans lequel ledit médicament comprend en outre un inhibiteur de l'enzyme de conversion de l'angiotensine I ou un antagoniste des récepteurs AT1.

**Claims**

**1.** Pharmaceutical composition, **characterised in that** it comprises as active substance at least (S) 3-amino-4-mercaptobutyl sulphonic acid or one of the salts thereof with a pharmaceutically acceptable acid or base for the use thereof as medication.

**2.** Composition for the use thereof according to claim 1, **characterised in that** it comprises furthermore at least one pharmaceutically acceptable vehicle.

**3.** Composition for the use thereof according to claim 1 or 2, **characterised in that** it comprises furthermore an angiotensin I-converting enzyme inhibitor or an AT1 receptor antagonist.

**4.** Selective inhibitor of aminopeptidase A which:

- has an affinity multiplied by at least a factor 100 for aminopeptidase A compared with aminopeptidase N, or
- has an inhibitory power over aminopeptidase A in vitro which is less than or equal to $10^{-7}$ M associated if necessary with a pharmaceutically acceptable vehicle, for the use thereof as medication for decreasing blood pressure.

**5.** Inhibitor for the use thereof according to claim 4, in which the inhibitor is (S) 3-amino-4-mercaptobutyl sulphonic acid or one of the salts thereof with a pharmaceutically acceptable acid or base.

**6.** Inhibitor for the use thereof according to claim 4 or 5, in which said medication comprises furthermore an angiotensin I-converting enzyme inhibitor or an AT1 receptor antagonist.

**Patentansprüche**

**1.** Pharmazeutische Zusammensetzung **dadurch gekennzeichnet, dass** sie als aktiven Bestandteil mindestens (S) 3-Amino-4-mercoptobutylsulfonsäure oder eines ihrer Salze mit einer pharmazeutisch verträglichen Säure oder Base zur Verwendung als Arzneimittel umfasst.

**2.** Zusammensetzung zur Verwendung gemäß Anspruch 1, **dadurch gekennzeichnet, dass** sie zusätzlich mindestens eine pharmazeutisch veträgliche Trägersubstanz umfasst.

**3.** Zusammensetzung zur Verwendung gemäß Anspruch 1 der 2, **dadurch gekennzeichnet, dass** sie zusätzlich einen Inhibitor des Enzyms zur Umsetzung von Angiotensin i oder einen Antagonisten der AT1 Rezeptoren umfasst.

**4.** Selektiver Aminopeptidase-A-Inhibitor, der:

- eine vielfache Affinität von mindestens Faktor 100 für die Aminopetidase A im Vergleich zur Aminopeptidase N besitzt, oder
- eine hemmende Wirkung über die Aminopeptidase A in vitro unterhalb oder gleich $10^7$ M besitzt, gegebenenfalls verbunden mit einer pharmazeutisch verträglichen Trägersubstanz zur Verwendung als Arzneimittel zur Senkung des Blutdruckes.

**5.** Inhibitor zur Verwerdung gemäß Anspruch 4, wobei der Inhibitor (S) 3-Amino-4-mercaptobutylsulfonsäure oder eines ihrer Salze mit einer pharmazeutisch verträglichen Säure oder Base ist.

**6.** Inhibitor zur Verwendung gemäß Anspruch 4 oder 5, wobei das Arzneimittel zusätzlich einen Inhibitor des Enzyms zur Umsetzung von Anglotensin I oder einen Antagonisten der AT1 Rezeptoren umfasst.

FIG.1

FIG.2

**_FIG.3_**

**_FIG.4_**

SHR

FIG.5

FIG.6

WKY

**FIG.7**

FIG.8

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **Zini et al.** *Proc. Natl. Acad. sci. USA,* 1996, vol. 93, 11968-11973
- **song et al.** *brain Research,* 1997, vol. 744, 1-6
- **Chauvel et al.** *J. Med. Chem.,* vol. 37, 1339-13462950-2957
- **Fournié-Zaluski et al.** *J. Med. Chem.,* vol. 35, 1259-1266
- **Haley ; McCormick.** *Br. J. Pharmacol.,* vol. 12, 12-15
- **Ledwith et al.** *Anal. Biochem.,* vol. 213, 349-355
- **Vazeux.** *J. biochem.,* 1996, vol. 271, 9069-9074
- **Chauvel.** *J. Med. Chem.,* 1994, vol. 37, 2950-2956
- **G. Paxinos ; C. Watson.** Atlas stéréotaxique. Academic Press, 1986